# EUROPÄISCHE PATENTSCHRIFT

(11) **EP 2 724 692 B1**
(45) Veröffentlichungstag und Bekanntmachung des Hinweises auf die Patenterteilung: **20.05.2015**
(21) Anmeldenummer: 12189674.0
(22) Anmeldetag: 24.10.2012
(51) Int. Cl.: A61F 2/46

(54) **Halter für ein medizinisches Implantat**
Holder for a medical implant
Support d'un implant médical

(43) Veröffentlichungstag der Anmeldung: 30.04.2014
(73) Patentinhaber: WALDEMAR LINK GmbH & Co. KG, 22339 Hamburg (DE)
(72) Erfinder: Dmuschewsky, Klaus, 22397 Hamburg (DE)
(74) Vertreter: Raffay & Fleck

(56) Entgegenhaltungen:
- EP-A1- 1 774 927
- WO-A1-2005/004757
- US-A1- 2010 211 119

## Beschreibung

Die vorliegende Erfindung betrifft einen Halter für ein medizinisches Implantat oder eine medizinische lmplantatkomponente, welcher Halter einen Griffabschnitt und einen Halteabschnitt aufweist.

Derartige Halter für medizinische Implantate sind an sich bekannt. Ein Haltes gemäß den Merkmalen des Oberbegriffs des Anspruchs 1 ist aus EP-A-1 774 927 bekannt. Ein Beispiel für einen Halter für ein medizinisches Implantat in Form einer künstlichen Gelenkpfanne (die insoweit auch als Implantatkomponente eines gesamten künstlichen Gelenkes aufgefasst werden kann) ist z.B. aus der WO 2005/122970 A1 bekannt. Dieser Halter weist einen Halteabschnitt auf, in welchem ein mittels eines Spannkonus spreizbares Andruckelement radial nach außen spreizbar ist. Dieses Andruckelement wird im Einsatz des Halters in eine Gelenkpfanne eingebracht und durch Spreizen mittels des Spreizkonus gegen die Wand der Gelenkpfanne gedrückt, so dass sie dort kraft-, ggf. auch formschlüssig, anliegt. Zum Verwenden dieses Halters weist dieser einen Griffabschnitt auf. Dieser vorbekannte Halter wird während eines operativen Eingriffes zum Ersetzen eines verschlissenen natürlichen Gelenkes eingesetzt. Dabei wird beispielsweise zunächst eine für den operativen Eingriff bereitgelegte und vorbereitete (insbesondere sterilisierte) Gelenkpfanne an dem Halteabschnitt des Halters befestigt, der Halter dient dann weiterhin dem richtigen Setzen der künstlichen Gelenkpfanne im Bereich einer für die Implantation vorbereiteten Stelle des Knochens, zudem auch dem Einschlagen, d.h. dem Eintreiben der Gelenkschale in die richtige Implantatposition im Knochen. Der Halter kann aber auch in umgekehrter Weise dazu verwendet werden, bei einer Revisionsoperation eine bereits am Knochen festgelegte Gelenkschale zu entfernen. Dazu wird der Halter mit seinem Halteabschnitt an einer solchen Gelenkschale festgelegt, und durch Aufbringen entsprechender Kräfte am Griffabschnitt wird die Gelenkschale aus der Implantationsstellung gelöst und kann dann zusammen mit dem Halter entnommen werden.

Solche Halter dienen also der Handhabung während eines operativen Eingriffes zum Setzen und/oder Entfernen von medizinischen Implantaten und/oder Implantatkomponenten. Sie können in dieser Funktion mithin auch als Setz-, Einsetz- bzw. Entnahmeinstrumente aufgefasst und angesehen werden. Gleichermaßen ist es aber auch möglich, derartige Halter im Bereich der vorbereitenden Behandlung eines medizinischen Implantates bzw. einer medizinischen Implantatkomponente zu verwenden, beispielsweise um ein solches Implantat bzw. eine solche Implantatkomponente während eines Sterilisationsvorganges definiert zu greifen und zu halten und/oder nach einem solchen Vorgang aus einem Sterilisationsbecken zu entnehmen.

Nun ist ein solcher vorbekannter Halter in seiner Verwendungsform sehr spezifisch und kann letztlich nur mit Gelenkpfannen oder anderen einen gewölbten Innenraum aufweisenden medizinischen Implantaten bzw. Implantatkomponenten verwendet werden. Andere Ausgestaltungen der medizinischen Implantate oder Implantatkomponenten sich mit einem solchen Halter nicht kompatibel, es müssen andere Halter bzw. Haltesysteme gefunden werden.

Hier Abhilfe zu schaffen und einen entsprechenden Halter anzugeben, der mit anderen medizinischen Implantaten oder Implantatkomponenten verwendet werden kann, ist Aufgabe der Erfindung. Diese Aufgabe wird erfindungsgemäß gelöst durch einen Halter für ein medizinisches Implantat oder eine medizinische lmplantatkomponente mit einem Griffabschnitt und einem Halteabschnitt, der weiterhin die kennzeichnenden Merkmale des Anspruches 1 aufweist bzw. verwirklicht. Vorteilhafte Weiterbildungen eines solchen Halters sind in den abhängigen Ansprüchen 2 bis 9 angegeben. In einem weiteren Aspekt besteht die Erfindung auch in einer Kombination, die gebildet ist aus einem neuartigen Halter und einem medizinischen Implantat und die näher in Anspruch 10 bezeichnet ist.

Erfindungsgemäß weist der Halter ein in dem Halteabschnitt angeordnetes Basiselement auf. Er hat ferner wenigstens zwei in dem Halteabschnitt voneinander beabstandet angeordnete Haltestifte, die sich ausgehend von einer Außenseite des Basiselementes in axialer Richtung jeweils bis zu einem freien Ende erstrecken. In dem Basiselement ist weiterhin wenigstens eine Aufnahmeöffnung vorgesehen, welche das Basiselement von einer dessen Außenseite gegenüberliegenden Innenseite bis zu der Außenseite durchdringt und sich über zumindest einen Abschnitt in Richtung der Außenseite kontinuierlich verjüngt. In dieser Aufnahmeöffnung ist wenigstens ein erster Haltestift so eingesetzt, dass er ein Spiel gegenüber der Aufnahmeöffnung, dies ist insbesondere ein quer zu der Längserstreckung des Haltestiftes wirkendes Spiel, aufweist. Dieser erste Haltestift, der in der Aufnahmeöffnung eingesetzt ist, verfügt dabei über einen axialen Abschnitt, in welchem er in Richtung des freien Endes, also desjenigen Endes, das über die Außenseite des Basiselementes hinaus vorsteht, einen sich verjüngenden Durchmesser aufweist. Der Haltestift weist ferner eine an seinem auf der Innenseite des Basiselementes liegenden Ende ausgebildete Druckfläche auf. Zudem sind einerseits der Abschnitt, in dem sich die Aufnahmeöffnung kontinuierlich verjüngt, und andererseits der axiale Abschnitt des ersten Haltestiftes mit dem sich verjüngenden Durchmesser derart gebildet, dass sie bei in der Aufnahmeöffnung sitzendem ersten Haltestift in ihrem Zusammenwirken bewirken, dass der erste Haltestift um einen durch dieses Zusammenwirken gebildeten Drehpunkt quer zu seiner Längsachse verschwenkbar ist. Mit anderen Worten lässt sich der erste Haltestift, der in der Aufnahmeöffnung eingesetzt ist, quer zu seiner axialen Richtung zumindest um ein gewisses Maß verschwenken und bewegen, so dass er unterschiedliche Winkelstellungen gegenüber der Oberfläche der Außenseite des Basiselementes einnehmen kann. Der erfindungsgemäße Halter weist schließlich noch ein in Richtung der Innenseite des Halteabschnittes verlagerbares Druckelement auf, welches auf einen einseitig einer Mittelachse des ersten Haltestiftes gelegenen, diese Mittelachse nicht überdeckenden Abschnitt der Druckfläche wirkt. Dieses Druckelement kann also ein außerhalb und einseitig der Mittelachse des ersten Haltestiftes wirkendes Moment aufbringen, welches sich dann als Kippmoment auswirkt und ein erzwungenes Verschwenken des Haltestiftes um den Drehpunkt bewirkt.

Mit diesem erfindungsgemäßen Halter kann ein medizinisches Implantat oder eine medizinische Implantatkomponente ergriffen und für ein Handling vor, während oder auch nach einer Operation mit dem Halter und somit mit einem Griffabschnitt verbunden werden. Hierzu ist es lediglich erforderlich, dass das medizinische Implantat bzw. die Implantatkomponente entsprechende Halteöffnungen aufweist, die in ihrer Anordnung relativ zueinander (also der Positionierung), ihrer Erstreckungsrichtung und ihren Abmessungen zu den entsprechenden Anordnungen (Positionierungen relativ zueinander) der Haltestifte, deren Verläufen (Erstreckungsrichtungen) sowie Abmessungen (insbesondere Durchmessern) korrespondieren. Dann nämlich kann der Halter mit dem Halteabschnitt, genauer mit den über die Außenseite des Basiselementes überstehenden Haltestiften in die entsprechenden Halteöffnungen an dem medizinischen Implantat bzw. der Implantatkomponente eingeführt werden, und durch Verlagern des Druckelementes in Richtung der Druckfläche des Haltestiftes und Aufbringen einer abseits der Mittelachse des ersten Haltestiftes liegenden Kraft kann dieser erste Haltestift um den Drehpunkt verkippt werden, so dass er in der zugehörigen Halteöffnung verklemmt. Das Druckelement wird dann in einer solchen Position festgelegt bzw. festgehalten, und das medizinische Implantat bzw. die Implantatkomponente ist fest und sicher mit dem Halter verbunden, kann beispielsweise am Implantatort im menschlichen Körper gesetzt werden, von dort entnommen werden und dgl.

Eine mögliche derartige Implantatkomponente kann beispielsweise ein typischerweise aus Kunststoff gebildetes Plateau eines künstlichen Knies, also die auf der Tibiakomponente oberseitig aufgesetzte Lauffläche für die Femurkomponente dieses künstlichen Gelenkes sein. Bei einem solchen Plateau lassen sich beispielsweise an einer Stirnseite problemlos entsprechende Halteöffnungen anbringen, so dass mit einem erfindungsgemäßen Halter dieses Plateau in der wie oben beschriebenen Weise klemmend ergriffen und gehalten und gesetzt werden kann. Selbstverständlich ist der Gebrauch eines solchen erfindungsgemäßen Halters nicht auf ein solches skizziertes Beispiel beschränkt, es kommen hier auch andere Implantatkomponenten in Betracht, die mit entsprechenden Halteöffnungen versehen werden können.

Ein solcher erfindungsgemäßer Halter ist nicht nur in seiner mechanischen Gestaltung besonders einfach und dabei zuverlässig in seiner Haltewirkung, er lässt sich auch in einer Weise realisieren, die eine besonders einfache Reinigung und Sterilisierung des typischerweise wieder verwendbaren Instrumentes ermöglicht. Dabei können zwar mehr als nur einer (der erste) Haltestift in einer zugeordneten Aufnahmeöffnung aufgenommen und entsprechend wie oben beschrieben verschwenkbar gebildet sein. Es hat sich aber herausgestellt, dass dies nicht erforderlich ist, sondern dass es in der Regel genügt, lediglich einen einzigen Haltestift in dieser Weise verschwenkbar zu gestalten. Damit können weitere Haltestifte als fest mit dem Basiselement verbundene Haltestifte, insbesondere an diesem einstückig angeformte Haltestifte, gebildet sein. Insbesondere bei einer einstückigen Anformung, ergeben sich keine Spalten oder sonstigen Übergänge, die in einer postoperativen Reinigung und Sterilisation problematisch, da schwer zu erreichen, sein können. Die Oberfläche an der Außenseite des Basiselementes, an der die Haltestifte hervortreten und über die diese bis zu ihrem freien Ende überstehen, kann dabei insbesondere so geformt sein, dass sie eine einfache Reinigung und Sterilisierung erlaubt. Sie kann insbesondere eben und flach ausgebildet sein. Sie kann aber auch an eine Oberfläche eines mit dem Halter zu ergreifenden Implantates bzw. einer Implantatkomponente angepasst sein, beispielsweise einen entsprechend invers gekrümmten Verlauf aufweisen.

Der in der Aufnahmeöffnung sitzende Haltestift kann zwar im Rahmen der Erfindung mit dem Material des Basiselementes noch über flexible Brücken verbunden sein. Insbesondere ist er aber frei aus der Aufnahmeöffnung entnehmbar, so dass er für eine Reinigung des erfindungsgemäßen Halters entnommen und einzeln gereinigt und sterilisiert werden kann. Der restliche Halter, insbesondere das restliche Basiselement kann gesondert gereinigt und sterilisiert werden, wobei dadurch, dass die Aufnahmeöffnung sich kontinuierlich verjüngt, auch dort idealer Weise keine in der Aufnahmeöffnung liegenden Absätze und Kanten gegeben sind, die für eine Reinigung und Sterilisierung schwer zugänglich sind.

Damit der lose von der Innenseite des Basiselementes der in die Aufnahmeöffnung eingesetzte Haltestift nicht in Richtung der Außenseite aus dieser heraus fällt, sind entsprechende Rückhaltestrukturen vorgesehen. Hierbei genügt es in einer bevorzugten Ausführungsform, die ohnehin miteinander zur Bildung des Drehlagers zusammenwirkenden Abschnitte (Strukturen) in der Aufnahmeöffnung und an dem Haltestift, in welchen sich die Aufnahmeöffnung und auch der Durchmesser des Haltestiftes jeweils verjüngen, für einen solchen Rückhalt zu nutzen. Denn neben einem Drehpunkt bilden diese Abschnitte bzw. Strukturen im Wechselspiel auch einen Rückhalt des ersten Haltestiftes und Anschlag gegen eine weitere Längsbewegung dieses ersten Haltestiftes in der Aufnahmeöffnung in Richtung der Außenseite des Basiselementes.

Besonders einfach lässt sich der erfindungsgemäße Halter verwenden, wenn die Haltestifte in ihrer jeweiligen axialen Richtung im Wesentlichen parallel verlaufen. Dabei ist unter diesem Verlauf insbesondere derjenige Verlauf zu verstehen, der sich im Bereich zwischen der Oberfläche des Basiselementes an der Außenseite bis hin zu den freien Enden der Haltestifte ergibt. Mit der Umschreibung "im Wesentlichen parallel" ist hierbei auch berücksichtigt, dass der erste, in der Aufnahmeöffnung in dem Basiselement sitzende Haltestift ein Verschwenken quer zu seiner axialen Richtung ermöglicht bzw. erfährt, so dass die Ausrichtung seiner axialen Richtung veränderlich ist. Gemeint ist hier, dass in einer möglichen Schwenkstellung des ersten Haltestiftes dessen Längsachse tatsächlich parallel ausgerichtet ist zu den Längsachsen der weiteren Haltestifte, seien diese feststehende oder ebenfalls in einer Aufnahmeöffnung verschwenkbar angeordnet. Eine solche parallele Ausrichtung erlaubt ein besonders einfaches Einführen der Haltestifte in entsprechend ebenfalls parallel ausgerichtete Halteöffnungen an dem medizinischen Implantat bzw. der medizinischen Implantatkomponente. Grundsätzlich kann der medizinische Halter mehrere Haltestifte aufweisen, insbesondere also mehr als zwei. Allerdings genügt es für viele Anwendungen, wenn er genau zwei Haltestifte aufweist, Insoweit wird eine solche Ausgestaltung auch bevorzugt. Denn einerseits müssen bei einer größeren Zahl von Haltestiften entsprechend mehr Halteöffnungen an dem medizinischen Implantat bzw. der Implantatkomponente vorgesehen werden, was die Implantatkomponente prinzipiell strukturell schwächen kann und zudem einen größeren Raum bzw. eine größere Fläche erfordert, in dem bzw. quer zu der die Halteöffnungen eingebracht sind. Zum anderen erfordern Haltestifte und damit in dem Implantat bzw. in der Implantatkomponente vorzusehende Halteöffnung in größerer Zahl ein höheres Maß an Maßhaltigkeit und verringern die zulässigen Toleranzen. In einer besonderen und einfachen, bevorzugten Ausgestaltung hat der erfindungsgemäße Halter genau zwei Haltestifte, von denen einer, nämlich der erste Haltestift in der zugeordneten Aufnahmeöffnung sitzt und entsprechend verschwenkbar ist, der andere, der zweite Haltestift, fest mit dem Basiselement verbunden ist. In dieser Ausgestaltung sind die beiden Haltestifte bevorzugt mit ihren Längsachsen im Wesentlichen parallel ausgebildet und ergeben somit eine Art "Haltegabel".

Mit Vorteil kann bei dem erfindungsgemäßen Halter der erste Haltestift mit seiner Druckfläche über die Oberfläche des Basiselementes überstehen. Bei einer solchen Ausgestaltung kann das Druckelement plan und eben ausgebildet sein und mit einem Teil gegen die Druckfläche, mit einem weiteren Teil gegen die Oberfläche des Basiselementes an dessen Innenseite drücken. Ist hingegen, was grundsätzlich auch möglich ist, die Druckfläche des ersten Haltestiftes in der Aufnahmeöffnung versenkt, muss das Druckelement dornartig in die Aufnahmeöffnung hineinragen, was insbesondere bei der Übertragung höherer Kräfte ungünstig ist, wie sie für die Umsetzung bzw. für das Aufbringen entsprechender Klemmkräfte auf eine in dem medizinischen Implantat bzw. in der Implantatkomponente ausgebildete Halteöffnung erforderlich ist. Hier ist eine breite und flächenartige auf die Druckfläche einwirkende Oberfläche des Druckelementes, wie sie bei einer über die Oberfläche des Basiselementes auf der Innenseite überstehenden Druckfläche des ersten Haltestiftes möglich ist, günstiger als ein dornartiges Druckelement, welches in die Aufnahmeöffnung in dem Basiselement eindringen und dort wirken muss.

Der Handgriff des erfindungsgemäßen Halters kann in einer vorteilhaften Ausgestaltung als in einer axialen Richtung langgestreckter, an der Innenseite des Basiselementes angeordneter Handgriff gebildet sein, wobei dann bevorzugt das Druckelement an einem der Innenseite des Basiselementes benachbarten Abschnitt des Handgriffes angeordnet und in Richtung der Innenseite des Basiselementes verlagerbar und in einer Zielposition festlegbar ist. Besonders einfach lässt sich dieses Zusammenwirken zwischen Druckelement und Handgriff erreichen, wenn an dem Handgriff ein Absatz gebildet ist, der in Richtung der Innenseite des Basiselementes vorsteht bzw. wirkt, und wenn ferner der Handgriff insgesamt mit dem Basiselement in axialer Richtung des Handgriffes verlagerbar verbindbar ist. Eine solche Verbindbarkeit kann insbesondere durch eine Schraubverbindung, beispielsweise ein Innengewinde in dem Basiselement und ein Außengewinde an dem Handgriff realisiert werden. So kann durch Einschrauben des Handgriffes in Richtung des Basiselementes, insbesondere in Richtung der Oberfläche auf dessen Innenseite der Absatz gegen die Druckfläche des ersten Haltestiftes gepresst und eine hohe Andruckkraft aufgebracht werden zum Erzeugen einer ausreichend großen Klemmkraft bei der verschwenkenden Verlagerung des ersten Haltestiftes.

Die Position, an der der Handgriff auf der Innenseite des Basiselementes festgelegt ist, an der also seine Längsachse die Oberfläche des Basiselementes schneidet, kann insbesondere auf einer Verbindungslinie der Schnittpunkte der Längsachsen von zwei Haltestiften mit der Oberfläche des Basiselementes auf dessen Innenseite liegen, bevorzugt dabei in der Mitte zwischen diesen Schnittpunkten. Insbesondere der Schnittpunkt der Längsachse eines in seiner axialen Richtung verschwenkbaren Haltestiftes mit der Oberfläche des Basiselementes auf seiner Innenseite ist bedingt durch die Verschwenkbarkeit in einem Rahmen variabel. Hierbei soll insbesondere derjenige Punkt als Schnittpunkt definiert sein, der sich bei einer bevorzugten Ausrichtung des Haltestiftes für das Einführen desselben in eine Halteöffnung an dem medizinischen Implantat bzw. an der Implantatkomponente einstellt. Insbesondere kann dies eine solche Ausrichtung sein, in der die Haltestifte in ihrer axialen Richtung zueinander parallel ausgerichtet liegen.

Eine solche Anordnung gibt einerseits eine symmetrische Anordnung, führt andererseits dazu, dass das Druckelement, welches entlang der Längserstreckungsachse des Handgriffes in Richtung der Oberfläche auf der Innenseite des Basiselementes verlagerbar ist, auf einer dem benachbarten Haltestift zugewandten Seite des ersten Haltestiftes angreift, diesen also bei Aufbringen einer Druckkraft mit seinem über die Außenseite überstehenden freien Ende nach außen, also von dem benachbarten Haltestift weg verschwenkt und damit eine nach außen gerichtete Klemmkraft aufbringt. Eine solche nach außen gerichtete Klemmkraft ist häufig eine für das zuverlässige Ergreifen und Halten günstigere.

Die Abschnitte, die in ihrer Zusammenwirkung einen Drehpunkt bilden, also derjenige Abschnitt, in dem sich die Aufnahmeöffnung kontinuierlich verjüngt und der axiale Abschnitt des ersten Haltestiftes, mit sich verjüngendem Durchmesser, können ganz unterschiedlich geformte sein. Beispielsweise können dies sphärisch gebildete Abschnitte sein mit einer teilsphärischen Aufnahme in der Aufnahmeöffnung und einer entsprechenden Teilsphäre an dem Haltestift, die in ihren Durchmessern aufeinander abgestimmt sind, um die beschriebene Bildung eines Drehpunktes zu erreichen. Es können aber die Abschnitte insbesondere auch jeweils sich konisch verjüngend ausgebildet sein. Dann müssen, um nicht einen exakten konischen und quer zur Längsachse des ersten Haltestiftes festgelegten Sitz zu erhalten, unterschiedliche Konuswinkel gewählt werden, insbesondere sollte der axiale Abschnitt des ersten Haltestiftes einen kleineren Konuswinkel aufweisen als der sich kontinuierlich verjüngende Abschnitt der Aufnahmeöffnung. So sitzt ein Abschnitt des Stiftes mit steilerer Flanke in einem Abschnitt der Aufnahme mit flacher abfallenden Wänden, was zu einer entsprechenden quer zur Längsrichtung des Haltestiftes wirkenden Bewegungsmöglichkeit, einem Drehpunkt, führt. Dabei ist im Sinne der Erfindung ein "Drehpunkt" nicht als ein solcher zu verstehen, der zwingend für die Schwenkbewegung fix liegt. Dieser kann auch wandern und seine Position während eines Bewegungsvorganges verändern. Entscheidend ist lediglich, dass eine Lagerung des Haltestiftes in einem mittleren Abschnitt erreicht wird durch das Zusammenwirken der konisch sich verjüngenden Abschnitte der Aufnahmeöffnung und des Haltestiftes, so dass das Aufbringen eines Kippmomentes auf den Haltestift an seinem mit der Druckfläche versehenen Ende in der einen Richtung ein Verkippen des freien Endes in eine entlang der ursprünglichen Ausrichtung seiner Längsachse gegenüberliegende Richtung bewirkt.

Weitere Vorteile und Merkmale der Erfindung ergeben sich aus der nachfolgenden Beschreibung eines Ausführungsbeispiels anhand der beigefügten Figuren. Dabei zeigen:
- Figur 1: eine perspektivische Ansicht eines Ausführungsbeispiel eines erfindungsgemäßen Halters in einer Ansicht schräge von vorn,
- Figur 2: in einer Explosionsdarstellung den erfindungsgemäßen Halter gemäß Figur 1 mit seinen einzelnen Komponenten,
- Figur 3: in zwei Darstellungen a) und b) den erfindungsgemäßen Halter gemäß Figur 1 in zwei unterschiedlichen Stellungen vor dem Spreizen der Haltestifte (Fig. 3a) und mit abgespreiztem Haltestift (Fig. 3b) zum Verklemmen eines mit dem Halter ergriffenen Implantats, und
- Figur 4: in einer weiteren Darstellung den erfindungsgemäßen Halter in der Ausführung gemäß Figur 1 im Zusammenwirken mit einem mit diesem ergriffenen und an ihm festgelegten medizinischen Implantat.

In den Figuren sind schematische Zeichnungen eines Ausführungsbeispiels für einen erfindungsgemäßen Halter dargestellt. Bei den Figuren handelt es sich um Prinzipskizzen zur Erläuterung des erfindungsgemäßen Ausführungsbeispiels insbesondere im Hinblick auf die erfindungswesentlichen Gestaltungsmerkmale. Diese Darstellungen sind jedoch keinesfalls maßstabsgerecht oder als vollwertige Konstruktionszeichnungen zu verstehen. Sie stellen ebenso lediglich ein Ausführungsbeispiel von mehreren möglichen Ausführungsvarianten eines erfindungsgemäßen Halters dar.

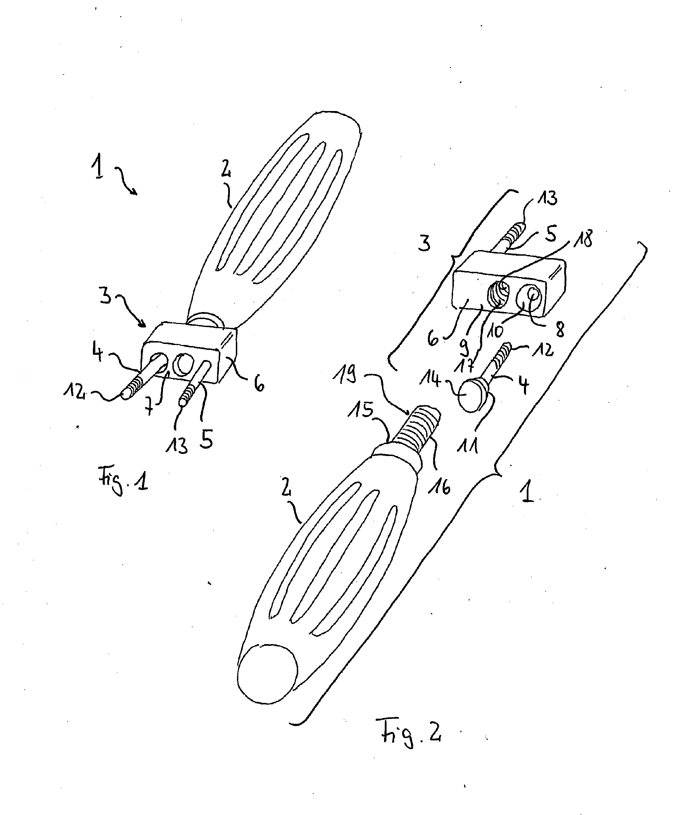

In den Figuren ist in unterschiedlichen Darstellungen ein erfindungsgemäßes Ausführungsbeispiel für einen Halter gezeigt und dort allgemein mit der Bezugsziffer 1 bezeichnet. Der Halter 1, der zunächst unter Bezugnahme auf die Figuren 1 und 2 im Grundsatz beschrieben wird, umfasst einen Griffabschnitt 2, in diesem Fall einen axial langgestreckten Handgriff, sowie einen Halteabschnitt 3, der sich in einem vorderen Bereich an den Griffabschnitt 2 anschließt. In dem Halteabschnitt 3 stehen auf einer dem Griffabschnitt 2 gegenüberliegenden Seite, der Außenseite 7, zwei Haltestifte 4 und 5 vor und enden in freien Enden 12 bzw. 13. Diese Haltestifte 4 und 5 sind an einem Basiselement 6 angeordnet bzw. festgelegt.

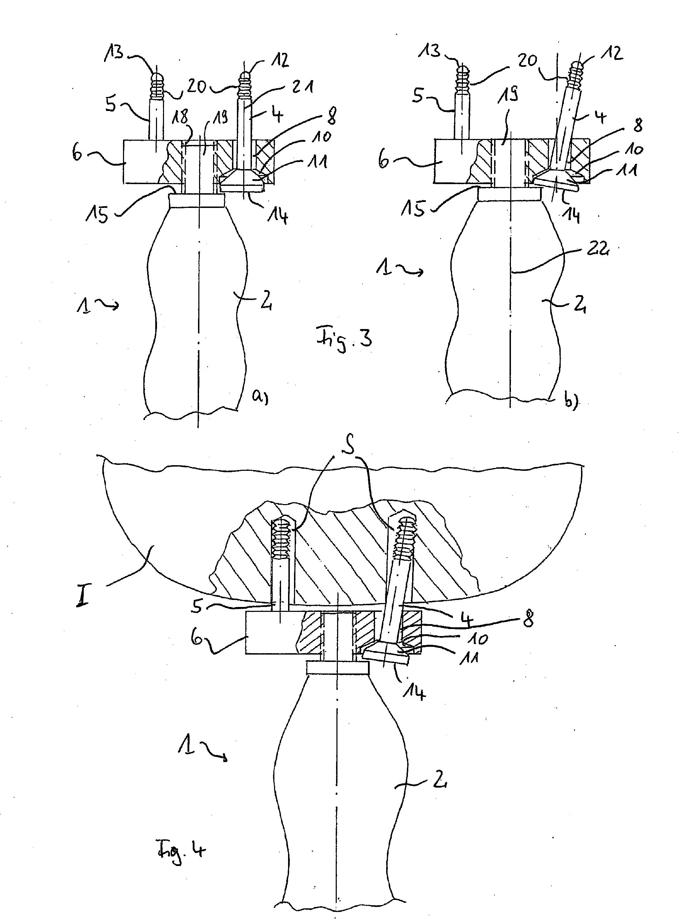

Wie insbesondere die Explosionsdarstellung gemäß Figur 2 erkennen lässt, sind die Haltestifte 4 und 5 unterschiedlicher Natur. Während der Haltestift 5 in diesem Ausführungsbeispiel einstückig mit dem Basiselement 6 gebildet und somit auf der Außenseite 7 fest angebracht ist mit in Bezug auf die Orientierung des Basiselementes 6 vorgegebener und nicht veränderbarer axialer Erstreckung, ist der Haltestift 4 lösbar in eine Aufnahmeöffnung 8 gesetzt, die das Basiselement 6 von einer Innenseite 9 bis zur Außenseite 7 durchdringt. In einem Abschnitt 10 ist die Aufnahmeöffnung 8 mit konisch sich in Richtung der Außenseite 7 verjüngendem Durchmesser ausgebildet. Dieser Abschnitt 10 beginnt in diesem Ausführungsbeispiel auf der Innenseite 9, wo die Aufnahmeöffnung 8 eine größeren Durchmesser hat, und erstreckt sich von da aus in Richtung der Außenseite 7 bis zu einem verjüngten Durchmesser der dann gerade weitergeführten Aufnahmeöffnung 8 (vergleiche hierzu auch Figuren 3a und 3b).

Der lösbar in dieser Aufnahmeöffnung 8 eingesetzte Haltestift 4 verfügt ebenfalls über einen Abschnitt 11 mit sich konisch verjüngendem Durchmesser. Er hat auf seiner dem freien Ende 12 gegenüberliegenden Seite zudem eine Druckfläche 14.

An seinem dem Basiselement 6 zugewandten Ende weist der Griffabschnitt 2 einen zapfenartigen Abschnitt 19 auf, der mit einem Außengewinde 16 versehen ist. Dieser Abschnitt 19 geht über einen Vorsprung 15, der eine Andruckfläche bildet, in den restlichen Griffabschnitt 2 über. Im Zentrum des Basiselementes 6 ist eine weitere Öffnung, die Schrauböffnung 18, ausgebildet und mit einem Innengewinde 17 versehen. Die Schrauböffnung 18 verläuft hier in ihrer axialen Erstreckung im Wesentlichen parallel zu der Aufnahmeöffnung 8. Beim Zusammenfügen des so aus insgesamt drei Einzelteilen, nämlich dem Griffabschnitt 2, dem Basiselement 6 mit daran angeformtem Haltstift 5 und dem lösbar in die Aufnahmeöffnung 8 eingesetzten Haltestift 4 gebildeten Halters 1 wird zunächst der Haltestift 4 in die Aufnahmeöffnung 8 eingesetzt, durch die er nicht zur Außenseite 7 des Basiselementes 6 hindurchrutschen kann aufgrund des Zusammenwirkens der Abschnitte 10 und 11. Sodann wird der zapfenartige Abschnitt 19 in die Schrauböffnung 18 eingesetzt und der Griffabschnitt 2 mit dem Basiselement 6 verschraubt.

In Figur 3 ist in den Darstellungen a und b die Situation dargestellt, wie sie sich beim letzten Wegrest des axialen Einschraubens des zapfenartigen 19 des Griffabschnittes 2 in die Schrauböffnung 18 ergibt. Dabei ist zunächst einmal zu erkennen, dass der sich verjüngende Abschnitt 11 des Haltestiftes 4 konisch gebildet ist, ebenso wie der sich verjüngende Abschnitt 10 der Aufnahmeöffnung 8. Dabei unterscheiden sich jedoch die Konuswinkel voneinander. Der Konuswinkel des Abschnittes 11 ist steiler als der Konuswinkel des Abschnittes 10, so dass der Abschnitt 11 und damit der Haltestift 4 sich in verkippender Weise gegenüber dem Abschnitt 10 und damit der Aufnahmeöffnung 8 bewegen kann.

Zudem ist zu erkennen, dass beim Einschrauben des zapfenartigen Abschnittes 19 in die Schrauböffnung 18 der Vorsprung 15 die Druckfläche 14 überragt in eine Bereich einseitig einer Mittelachse 21 des Haltestiftes 4. Wird nun ausgehend von der Figur 3a das Griffstück 2 mit seinem zapfenartigen Abschnitt 19 weiter in die Schrauböffnung 18 eingeschraubt, so stößt der Vorsprung 15 an dem Griffstück 2 weiter auf die Druckfläche 14 des Haltestiftes 4 und treibt, ermöglicht durch dessen Spiel in der Aufnahmeöffnung 8 sowie durch die unterschiedlich geformten Konuswinkel in den Abschnitten 11 und 10, den Haltestift 4 mit seinem freien Ende 12 ausgehend von einer Mittelachse 22 des Halters 1 nach außen.

In Figur 4 ist gezeigt, wie diese technische Wirkung zum Erfassen und Verklemmen eines medizinischen Implantates I ausgenutzt wird. In dem Implantat I sind passend zum Abstand der Stifte 4 und 5 und entsprechend deren in einer Ruhestellung im Wesentlichen parallelen Längsausrichtung Sacklochbohrungen S angebracht. In diese Sacklochbohrungen S werden die Haltestifte 4, 5 in einem Zustand eingeführt, wie er in Figur 3a gezeigt ist. Sodann wird durch weiteres Einschrauben des Griffabschnittes 2 mit seinem zapfenartigen Abschnitt 19 in die Schrauböffnung 18 und durch das damit bewirkte Zusammenwirken des Vorsprunges 15 mit der Druckfläche 14 der Haltestift 4, wie in Figur 3b gezeigt und anhand dieser Darstellung beschrieben, nach außen verkippt und gezwungen. Dadurch verklemmt der Halter 1 seine Haltestifte 4, 5 in den Sacklochbohrungen S, und das medizinische Implantat I ist sicher an dem Halter 1 befestigt und von diesem gehalten. Zu einer Erhöhung der Haltekraft sind im Bereich der freien Enden 12, 13 der Haltestifte 4, 5 Riffelungen 20 eingebracht.

Mit dem so an dem Halter 1 befestigten medizinischem Implantat I, z.B. einem aus Kunststoff, beispielsweise PE, bestehenden Plateau für die Tibiakomponente eines künstlichen Kniegelenkes, kann dieses Implantat zu seinem Implantationsort bewegt und dort platziert sowie bis zu seiner Verankerung gehalten werden. Ist das Implantat I dann sicher angebracht, wird durch Herausschrauben des Griffabschnittes 2 mit seinem zapfenartigen Abschnitt 19 aus der Schrauböffnung 18 der Kontakt zwischen dem Vorsprung 15 und der Druckfläche 14 gelöst, so dass der Haltestift 4 in Richtung seiner ursprünglichen Ausrichtung, in der er mit seiner Längsachse im Wesentlichen parallel zur Längsache des Haltestiftes 5 verläuft, zurückkippen kann und die Verklemmung gelöst wird. Dann kann der Halter 1 entnommen werden. In gleicher Weise kann der Halter 1 natürlich verwendet werden, um ein Implantat I im Rahmen einer Revisionsoperation von einem Implantationsort zu entfernen. Dann wird an dem noch in der Implantationsstellung befindlichen Implantat I der Halter 1 wie oben beschrieben befestigt, das Implantat I wird gelöst und entnommen und nach Entnahme wird der Halter 1, wie oben beschrieben, vom Implantat I gelöst.

Nach einem Einsatz kann der erfindungsgemäße Halter besonders einfach gereinigt und desinfiziert werden, da er aus lediglich drei Teilen, nämlich dem Griffabschnitt 2, dem Basiselement 6 mit daran angeformtem Haltestift 5 sowie dem lösbar in der Aufnahmeöffnung 8 sitzenden Haltestift 4 besteht. Alle Teile sind ohne schwer zugängliche Rücksprünge gebildet, so dass sie für Reinigungswerkzeuge wie etwa Bürsten einfach zu erreichen sind, keine Rückstände von Verunreinigungen zu befürchten sind.

### Bezugszeichenliste

- 1: Halter
- 2: Griffabschnitt
- 3: Halteabschnitt
- 4: Haltestif
- 5: Haltestift
- 6: Basiselement
- 7: Außenseite
- 8: Aufnahmeöffnung
- 9: Innenseite
- 10: Abschnitt
- 11: Abschnitt
- 12: freies Ende
- 13: freies Ende
- 14: Druckfläche
- 15: Vorsprung
- 16: Außengewinde
- 17: Innengewinde
- 18: Schrauböffnung
- 19: zapfenartiger Abschnitt
- 20: Riffelung
- 21: Mittelachse
- 22: Mittelachse

- I: Implantat
- S: Sacklochbohrung

## Patentansprüche

1. Halter für ein medizinisches Implantat (1) oder eine medizinische Implantatkomponente mit einem Griffabschnitt (2) und einem Halteabschnitt (3), wobei der Halter
a. ein in dem Halteabschnitt (3) angeordnetes Basiselement (6) aufweist, sowie
b. wenigstens zwei in dem Halteabschnitt (3) voneinander beabstandet angeordnete Haltestifte (4, 5), die sich ausgehend von einer Außenseite (7) des Basiselements (6) in axialer Richtung jeweils bis zu einem freien Ende (12, 13) erstrecken; **gekennzeichnet durch**
c. wenigstens eine Aufnahmeöffnung (8) in dem Basiselement (6), welche das Basiselement (6) von einer dessen Außenseite (7) gegenüberliegenden Innenseite (9) bis zu der Außenseite (7) durchdringt und sich über zumindest einen Abschnitt (10) in Richtung der Außenseite (7) kontinuierlich verjüngt,
- wobei wenigstens ein erster Haltestift (4) als in der Aufnahmeöffnung (8) mit einem Spiel eingesetzter Haltestift (4) mit einem axialen Abschnitt (11) ausgebildet ist, in welchem er in Richtung des freien Endes (12) einen sich verjüngenden Durchmesser aufweist,
- wobei der erste Haltestift (4) ferner eine an seinem auf der Innenseite (9) des Basiselements (6) liegenden Ende ausgebildete Druckfläche (14) aufweist,
- wobei der Abschnitt (10), in dem sich die Aufnahmeöffnung (8) kontinuierlich verjüngt, und der axiale Abschnitt (11) des ersten Haltestiftes (4) mit dem sich verjüngenden Durchmesser derart gebildet sind, dass, wenn der erste Haltestift (4) in der Aufnahmeöffnung (8) sitzt, dieser um einen **durch** Zusammenwirken des sich kontinuierlich verjüngenden Abschnittes (10) der Aufnahmeöffnung (8) und des sich kontinuierlich im Durchmesser verjüngenden axialen Abschnitts (11) des ersten Haltestiftes (4) gebildeten Drehpunkt quer zu seiner Längsachse verschwenkbar ist, und
d. ein in Richtung der Innenseite (9) des Haltabschnitts (3) verlagerbares Druckelement (1 5), welches auf einen einseitig einer Mittelachse (21) des ersten Haltestiftes (4) gelegenen, diese Mittelachse (21) nicht überdeckenden Abschnitt der Druckfläche (14) wirkt.

2. Halter nach Anspruch 1, **dadurch gekennzeichnet, dass** die wenigstens zwei Haltestifte (4, 5) in ihrer jeweiligen axialen Richtung im Wesentlichen parallel verlaufen.

3. Halter nach einem der vorhergehenden Ansprüche, **dadurch gekennzeichnet, dass** zumindest ein zweiter Haltestift (5) fest mit dem Basiselement (6) verbunden ist, insbesondere einstückig mit diesem gebildet ist.

4. Halter nach einem der vorhergehenden Ansprüche **gekennzeichnet durch** genau zwei Haltestifte (4, 5).

5. Halter nach einem der vorhergehenden Ansprüche, **dadurch gekennzeichnet, dass** der erste Haltestift (4) mit seiner Druckfläche (14) über die Oberfläche des Basiselements (6) übersteht.

6. Halter nach einem der vorhergehenden Ansprüche, **dadurch gekennzeichnet, dass** der Griffabschnitt (2) als in einer axialen Richtung langgestreckter, an der Innenseite (9) des Basiselements (6) angeordneter Handgriff gebildet ist und dass das Druckelement (15) an einem der Innenseite des Basiselementes (6) benachbarten Abschnitt des Handgriffs angeordnet und in Richtung der Innenseite des Basiselements (6) verlagerbar und in einer Zielposition festlegbar ist.

7. Halter nach Anspruch 6, **gekennzeichnet, durch** einen Absatz an dem Handgriff als Druckelement (15) und ferner **dadurch** gekennzeichnet, dass der Handgriff mit dem Basiselement (6) in axialer Richtung des Handgriffs verlagerbar verbindbar ist, insbesondere über eine Schraubverbindung (16, 17).

8. Halter nach einem der Ansprüche 5 oder 6, **dadurch gekennzeichnet**, das der Handgriff an einer Position auf der Innenseite (9) des Basiselements (6) festgelegt ist, die auf einer Verbindungslinie der Schnittpunkte der Längsachsen zweier Haltestifte (4, 5) mit der Oberfläche des Basiselements (6) auf dessen Innseite (9) liegt und in der Mitte zwischen diesen Schnittpunkten.

9. Halter nach einem der vorstehenden Ansprüche, **dadurch gekennzeichnet, dass** der Abschnitt (10), in dem sich die Aufnahmeöffnung (8) kontinuierlich verjüngt, und der axiale Abschnitt (11) des ersten Haltestiftes (4) mit sich verjüngendem Durchmesser sich jeweils konisch verjüngen, wobei der axiale Abschnitt (11) des ersten Haltestiftes (4) einen kleineren Konuswinkel aufweist als der sich kontinuierlich verjüngende Abschnitt (10) der Aufnahmeöffnung (8).

10. Kombination bestehend aus einem Halter (1) nach einem der vorhergehenden Ansprüche und einem medizinischen Implantat (1) oder einer medizinischen Implantatkomponente, wobei an dem Implantat (I) bzw. der Implantatkomponente in einem Ansetzabschnitt Halteöffnungen (S) vorgesehen sind, die in ihrer Positionierung relativ zueinander, ihrer Größe und ihrem Verlauf zu den Positionierungen der Haltestifte (4, 5) relativ zueinander, zu deren Größen und Verläufen korrespondieren und in die die Haltestifte (4, 5) einführbar sind.

## Claims

1. Holder for a medical implant (I) or a medical implant component, with a gripping portion (2) and a holding portion (3), which holder has
a. a base element (6) arranged in the holding portion (3), and also
b. at least two holding pins (4, 5), which are arranged spaced apart from each other in the holding portion (3) and, from an outer face (7) of the base element (6), each extend in the axial direction as far as a free end (12, 13);
**characterized by**
c. at least one receiving opening (8) in the base element (6), which receiving opening (8) passes through the base element (6) from an inner face (9), lying opposite the outer face (7) thereof, to the outer face (7) and tapers continuously in the direction of the outer face (7) over at least a portion (10),
- wherein at least a first holding pin (4) is designed as a holding pin (4) inserted with play in the receiving opening (8) and having an axial portion (11) in which it has a tapering diameter in the direction of the free end (12),
- wherein the first holding pin (4) moreover has a pressure surface (14) formed on its end lying on the inner face (9) of the base element (6),
- wherein the portion (10), in which the receiving opening (8) continuously tapers, and the axial portion (11) of the first holding pin (4) with the tapering diameter are formed in such a way that, when the first holding pin (4) sits in the receiving opening (8), it is pivotable, transversely with respect to its longitudinal axis, about a rotation point formed by interaction of the continuously tapering portion (10) of the receiving opening (8) and the axial portion (11) of continuously tapering diameter of the first holding pin (4), and
d. a pressure element (15), which is movable in the direction of the inner face (9) of the holding portion (3) and which acts on a portion of the pressure surface (14) that is located to one side of a central axis (21) of the first holding pin (4) and does not cover this central axis (21).

2. Holder according to Claim 1, **characterized in that** the at least two holding pins (4, 5) extend substantially parallel in their respective axial direction.

3. Holder according to one of the preceding claims, **characterized in that** at least a second holding pin (5) is connected fixedly to the base element (6), in particular formed in one piece with the latter.

4. Holder according to one of the preceding claims, **characterized by** precisely two holding pins (4, 5).

5. Holder according to one of the preceding claims, **characterized in that** the first holding pin (4) protrudes with its pressure surface (14) beyond the surface of the base element (6).

6. Holder according to one of the preceding claims, **characterized in that** the gripping portion (2) is formed as a handle which is elongate in an axial direction and is arranged on the inner face (9) of the base element (6), and **in that** the pressure element (15) is arranged on a portion of the handle adjacent to the inner face of the base element (6) and is movable in the direction of the inner face of the base element (6) and can be secured in a target position.

7. Holder according to Claim 6, **characterized by** a shoulder on the handle as pressure element (15) and further **characterized in that** the handle can be connected to the base element (6), in particular via a screw connection (16, 17), so as to be movable in the axial direction of the handle.

8. Holder according to either of Claims 5 and 6, **characterized in that** the handle is secured at a position, on the inner face (9) of the base element (6), which lies on a connecting line of the points of intersection of the longitudinal axes of two holding pins (4, 5) with the surface of the base element (6) on the inner face (9) thereof and in the centre between these points of intersection.

9. Holder according to one of the preceding claims, **characterized in that** the portion (10), in which the receiving opening (8) continuously tapers, and the axial portion (11) of tapering diameter of the first holding pin (4) each taper conically, wherein the axial portion (11) of the first holding pin (4) has a smaller cone angle than the continuously tapering portion (10) of the receiving opening (8).

10. Combination of a holder (1) according to one of the preceding claims and of a medical implant (I) or a medical implant component, wherein holding openings (S) are provided on the implant (I) or the implant component in an attachment portion, which holding openings (S) correspond, in terms of their position relative to each other, in terms of their size and in terms of their profile, to the positions of the holding pins (4, 5) relative to each other, and to the sizes and profiles of the holding pins (4, 5), and into which openings the holding pins (4, 5) can be inserted.

## Revendications

1. Support pour un implant médical (I) ou pour un composant d'implant médical comprenant une portion de préhension (2) et une portion de retenue (3), le support présentant
a. un élément de base (6) disposé dans la portion de retenue (3) et
b. au moins deux goupilles de retenue (4, 5) disposées à distance les unes des autres dans la portion de retenue (3), lesquelles s'étendent à partir d'un côté extérieur (7) de l'élément de base (6) dans la direction axiale à chaque fois jusqu'à une extrémité libre (12, 13) ;
**caractérisé par**
c. au moins une ouverture de réception (8) dans l'élément de base (6), qui traverse l'élément de base (6) depuis un côté intérieur (9) opposé à son côté extérieur (7) jusqu'au côté extérieur (7) et se rétrécit en continu dans la direction du côté extérieur (7) sur au moins une portion (10),
- au moins une première goupille de retenue (4) étant réalisée sous forme de goupille de retenue (4) insérée avec jeu dans l'ouverture de réception (8) avec une portion axiale (11), dans laquelle elle présente, dans la direction de l'extrémité libre (12), un diamètre se rétrécissant,
- la première goupille de retenue (4) présentant en outre une surface de pression (14) réalisée au niveau de son extrémité située sur le côté intérieur (9) de l'élément de base (6),
- la portion (10) dans laquelle l'ouverture de réception (8) se rétrécit en continu, et la portion axiale (11) de la première goupille de retenue (4) avec le diamètre se rétrécissant, étant réalisées de telle sorte que lorsque la première goupille de retenue (4) repose dans l'ouverture de réception (8), elle puisse pivoter transversalement à son axe longitudinal autour d'un centre de rotation formé par coopération de la portion (10) de l'ouverture de réception (8) se rétrécissant en continu et de la portion axiale (11) de la première goupille de retenue (4) dont le diamètre se rétrécit en continu, et
d. un élément de pression (15) pouvant être déplacé dans la direction du côté intérieur (9) de la portion de retenue (3), lequel agit sur une portion de la surface de pression (14) disposée d'un côté d'un axe médian (21) de la première goupille de retenue (4), et ne recouvrant pas cet axe médian (21).

2. Support selon la revendication 1, **caractérisé en ce que** les au moins deux goupilles de retenue (4, 5) s'étendent essentiellement parallèlement dans leur direction axiale respective.

3. Support selon l'une quelconque des revendications précédentes, **caractérisé en ce qu'**au moins une deuxième goupille de retenue (5) est connectée fixement à l'élément de base (6), en particulier est réalisée d'une seule pièce avec celui-ci.

4. Support selon l'une quelconque des revendications précédentes, **caractérisé par** exactement deux goupilles de retenue (4, 5).

5. Support selon l'une quelconque des revendications précédentes, **caractérisé en ce que** la première goupille de retenue (4) fait saillie avec sa surface de pression (14) au-delà de la surface de l'élément de base (6).

6. Support selon l'une quelconque des revendications précédentes, **caractérisé en ce que** la portion de préhension (2) est réalisée sous forme de poignée allongée dans la direction axiale, disposée sur le côté intérieur (9) de l'élément de base (6) et **en ce que** l'élément de pression (15) est disposé au niveau d'une portion de la poignée adjacente au côté intérieur de l'élément de base (6) et peut être déplacé dans la direction du côté intérieur de l'élément de base (6) et peut être fixé dans une position cible.

7. Support selon la revendication 6, **caractérisé par** un épaulement au niveau de la poignée, servant d'élément de pression (15) et **caractérisé en outre en ce que** la poignée peut être connectée à l'élément de base (6) de manière déplaçable dans la direction axiale de la poignée, en particulier par une connexion vissée (16, 17).

8. Support selon l'une quelconque des revendications 5 ou 6, **caractérisé en ce que** la poignée est fixée sur le côté intérieur (9) de l'élément de base (6) dans une position qui est située sur une ligne de connexion des points d'intersection des axes longitudinaux de deux goupilles de retenue (4, 5) avec la surface de l'élément de base (6) sur son côté intérieur (9), et au milieu entre ses points d'intersection.

9. Support selon l'une quelconque des revendications précédentes, **caractérisé en ce que** la portion (10) dans laquelle l'ouverture de réception (8) se rétrécit en continu et la portion axiale (11) de la première goupille de retenue (4) de diamètre se rétrécissant se rétrécissent à chaque fois sous forme conique, la portion axiale (11) de la première goupille de retenue (4) présentant un angle de conicité plus petit que la portion (10) de l'ouverture de réception (8) se rétrécissant en continu.

10. Combinaison constituée d'un support (1) selon l'une quelconque des revendications précédentes et d'un implant médical (I) ou d'un composant d'implant médical, des ouvertures de retenue (S) étant prévues au niveau de l'implant (I) ou du composant d'implant dans une portion d'application, lesquelles ouvertures de retenue, dans leur positionnement les unes par rapport aux autres, leur taille et leur allure, correspondent au positions des goupilles de retenue (4, 5) les unes par rapport aux autres, à leur taille et à leur allure, et peuvent être introduites dans les goupilles de retenue (4, 5).
